# EUROPEAN PATENT APPLICATION

(11) **EP 4 501 283 A1**
(43) Date of publication of application: **05.02.2025**
(21) Application number: 23779028.2
(22) Date of filing: 20.02.2023
(51) Int. Cl.: A61C 19/00, A61B 1/24

(54) **INTRAORAL NOTIFICATION DEVICE AND INTRAORAL MEDICAL INSTRUMENT**

(30) Priority: 31.03.2022 JP 2022059607
(71) Applicant: GC Corporation, Sunto-gun, Shizuoka 410-1307 (JP)
(72) Inventor: YANO, Seiji, Tokyo 174-8585 (JP); ISHIKAWA, Masaki, Tokyo 174-8585 (JP); YOSHIKAWA, Toshiya, Tokyo 174-8585 (JP)
(74) Representative: Müller-Boré & Partner Patentanwälte PartG mbB
(86) International application number: PCT/JP2023/005989
(87) International publication number: WO 2023/188998

(57) **Abstract**

An intraoral reporting device to report a state of an intraoral medical device, the intraoral reporting device including: an information processing unit; and a light source connected to the information processing unit, and arranged so as to be able to emit light in an oral cavity, wherein the information processing unit executes the process such that the light source is on, or on and off when detecting that the intraoral medical device is in a state where a function of the intraoral medical device is difficult to be maintained, or where the function thereof is difficult to be maintained soon.

## Description

### [Technical Field]

The present disclosure relates to reporting devices used for intraoral medical devices.

### [Background Art]

A medical device for intraoral use, such as a digital impression device to take intraoral impressions as digital data, has an intraoral scanner to three-dimensionally scan intraoral shapes. An operator inserts the tip of the intraoral scanner into an oral cavity and moves the scanner so that the tip moves just above and along dentition to scan the shape.

Patent literature 1 discloses that the statuses of a device are associated with light emission patterns to report by the use of the light emission patterns.

### [Citation List]

### [Patent Literature]

[Patent Literature 1] JP 2021-006385 A

### [Summary of Invention]

### [Technical Problem]

Intraoral scanning with a digital impression device as described above may lapse into a stopped state when processing scanned 3-D shape data does not follow the scanning because of a wet condition of, for example, dentition due to saliva: the shape of the dentition is being scanned, the speed of moving an intraoral scanner, etc. In order to continue the scanning, the intraoral scanner has to be returned to a position where the scanning is over. Generally, it is displayed on a screen such as a monitor that scanning is stopped. However, the operator is carefully observing a patient's oral cavity, and thus, may continue the operation while the scanning is being stopped without noticing the display on the screen.

Such a problem is not only for digital impression devices, but is common in intraoral medical devices.

An object of the present disclosure is to enable an operator to more certainly grasp the state of a medical device inserted into an oral cavity.

### [Solution to Problem]

The present application discloses an intraoral reporting device to report a state of an intraoral medical device, the intraoral reporting device comprising: an information processing unit; and a light source connected to the information processing unit, and arranged so as to be able to emit light in an oral cavity, wherein the information processing unit executes the process such that the light source is on, or on and off when detecting that the intraoral medical device is in a state where a function of the intraoral medical device is difficult to be maintained, or where the function thereof is difficult to be maintained soon.

An intraoral medical device comprising the above-described intraoral reporting device is also disclosed.

### [Advantageous Effects of Invention]

According to the present disclosure, an operator can cognize the state of the operation of an intraoral medical device without shifting their gaze from a patient's oral cavity when the state is reported, and thus, can more certainly grasp the state of the device.

### [Brief Description of Drawings]

[Fig. 1] Fig. 1 illustrates the structure of an intraoral medical device provided with an intraoral reporting device.
[Fig. 2] Fig. 2 illustrates operation of an intraoral reporting device.

### [Description of Embodiments]

Hereinafter the present disclosure will be described based on the embodiments shown in the drawings. The present disclosure is not limited to this embodiments. Here, a digital impression device will be described as one example of intraoral medical devices. This digital impression device includes an intraoral reporting device.

### [Digital Impression Device]

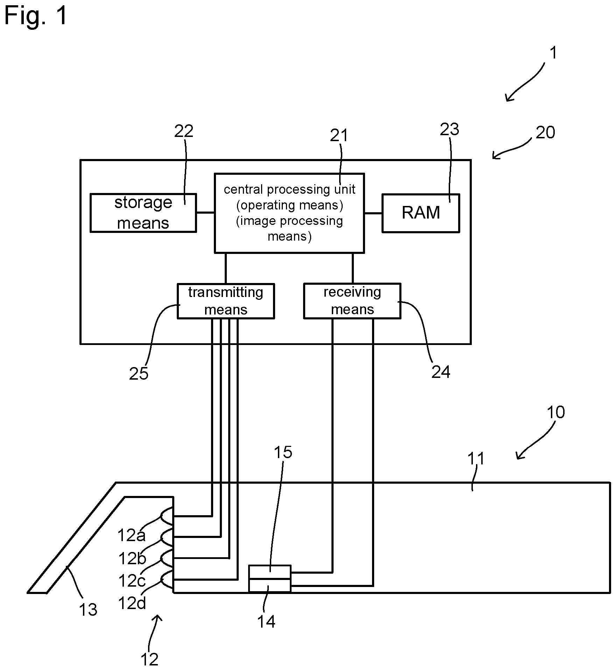

Fig. 1 schematically shows the structure of a digital impression device 1. As can be seen from Fig. 1, the digital impression device 1 is configured to have an intraoral scanner 10 and an information processing unit 20. The intraoral reporting device is also included here.

### <Intraoral Scanner>

The intraoral scanner 10 is the member to be partially inserted in an oral cavity and moved so that the part thereof moves over an area to obtain images: impressions of the area are to be taken. Therefore, the intraoral scanner 10 is provided with a main body 11, light sources 12, a reflecting mirror 13, a camera module 14, and a sensor 15.

The main body 11 holds each component of the intraoral scanner 10 and is a rod-like member that is held and operated by the operator.

The light sources 12 are arranged in the vicinity of a tip of the rod-like main body 11, and illuminate an oral cavity, so that intraoral images can be appropriately obtained. In this embodiment, LEDs are used as the light sources. More specifically, a white LED 12a, a red LED 12b, a green LED 12c, and a blue LED 12d are arranged.

The reflecting mirror 13 is a reflector arranged in the vicinity of the tip of the rod-like main body 11, and is disposed in such a way that the direction of the light emitted from the light sources 12 changes to the direction orthogonal to the axis of the main body 11. Thus, as shown in Fig. 1, the reflecting mirror 13 is arranged so as to face the light sources 12 and incline. This allows an object (such as a tooth) to be appropriately lighted with the light sources 12 so that efficient illumination can be provided: impressions of the object are to be taken.

The camera module 14 is provided in the vicinity of the tip of the rod-like main body 11 on a side face of the main body 11 which has a rod-like profile to take images of an area when the intraoral scanner 10 is inserted into an oral cavity: impressions of the area are to be taken. The camera module 14 enables light that comes from the oral cavity and that is an image to be taken to appropriately enter. In the camera module 14, the entered light is converted into electric signals. The converted signals are transmitted to the information processing unit 20 through a lead. A specific form of the camera module 14 is not particularly limited, and an example thereof is a device having a lens, and a photoelectric conversion sensor such as a CCD and a CMOS.

The sensor 15 is arranged adjacent to the camera module 14, and measures the temperature of the camera module 14. The type of the sensor 15 is not particularly limited as long as the sensor 15 allows the obtained temperature to be acquired as digital data.

### <Information Processing Unit>

The information processing unit 20 obtains the digital data from the camera module 14 and the sensor 15 of the intraoral scanner 10, and carries out necessary operations to process the images, and according to the results of the operations, sends the light sources 12 commands such as on, off, and on and off. Thus, the information processing unit 20 is configured to be provided with a central processing unit 21, a storage means 22, RAM 23, a receiving means 24, and a transmitting means 25.

The central processing unit 21 is what is called a CPU, and functions as an image processing means and any other various operating means. That is, the central processing unit 21 executes the various programs stored in the storage means 22 that functions as a storage medium, carries out operation to output certain results, and controls the digital impression device 1.

When functioning as an image processing means, the central processing unit 21 takes in the digital data from the camera module 14 via the receiving means 24, carries out operation based on any of the programs, and generates 3D image signals as a result of the operation.

When controlling the light sources 12, the central processing unit 21 makes each necessary light source on, off, or on and off as necessary. Specific control for the light sources will be described later.

When obtaining the temperature information from the sensor 15, the central processing unit 21 carries out operation based on the obtained temperature to control the digital impression device 1. The control based on the temperature will be also described later.

Other than the above, the central processing unit 21 is connected to any other components provided for the information processing unit 20 such as the storage means 22, the RAM 23, the receiving means 24, and the transmitting means 25, and is configured so as to be able to control them based on any of the programs.

The storage means 22 is the component that functions as a storage medium where various programs and databases that are the bases of the operation carried out in the central processing unit 21 are stored. The storage means 22 may further function as an archive means where data on images and patients etc. to be archived are archived.

The RAM 23 is the component that functions as a work area for the operation by the central processing unit 21, and as a temporary data storage means. The RAM 23 can be configured by SRAM, DRAM, flash memory, or the like, which is the same as a known RAM.

The receiving means 24 is the component having the function of taking in the electric signals from the camera module 14, which are based on the light from an oral cavity, and is connected to the camera module 14. The receiving means 24 also includes what one calls an input port, an input connector, etc. It does not matter whether the mode of the connection is wired or wireless.

The receiving means 24 is also connected to the sensor 15 electrically, which enables the information obtained in the sensor 15 to be taken into the information processing unit 20.

The transmitting means 25 is connected to at least the light sources 12, which enables the light sources 12 to be controlled to be on, etc. based on the operation results obtained by the information processing unit 20. When some obtained result should be transmitted to a display means such as a monitor (not shown), the display means is connected to the transmitting means 25, and signals are transmitted to the display means.

The transmitting means 25 also includes what one calls an output port, an output connector, etc. It does not matter whether the mode of the transmission is wired or wireless.

### <Intraoral Reporting Device>

An intraoral reporting device is the device for indicating, in an oral cavity, the operating state of an intraoral medical device provided therewith, and in this embodiment, indicates the operating state of the digital impression device 1, which is one intraoral medical device, in an oral cavity. In this embodiment, the intraoral reporting device has the information processing unit 20, the light sources 12, and the sensor 15. In this embodiment, the members and components constituting the intraoral reporting device are common to the intraoral scanner 10 and the information processing unit 20, and thus, the same signs are added thereto.

The information processing unit 20 in the intraoral reporting device obtains the state of the intraoral medical device, carries out operation to determine information to be reported, and sends commands to a reporting means (light sources).

The central processing unit 21 executes the programs stored in the storage means 22 that functions as a storage medium: the programs are for obtaining the state of the intraoral medical device, for determining a report, and for the commands to the reporting means (light sources). In this embodiment, when controlling the light sources 12, the central processing unit 21 makes each necessary light source on, off, or on and off as necessary. The light sources to be on etc. will be specifically described later.

The storage means 22 is the component that functions as a storage medium where various programs and databases that are the bases of the operation carried out in the central processing unit 21 are stored. Here, an example of the databases is a database where each state of the device is associated with any type of the light sources to be on, off, and on and off.

The RAM 23 is the component that functions as a work area for the operation by the central processing unit 21, and a temporary data storage means. The RAM 23 can be configured by SRAM, DRAM, flash memory, etc., which is the same as a known RAM.

The receiving means 24 is the component electrically connected to the sensor 15, and having the function of taking the information obtained in the sensor 15 into the information processing unit 20. The receiving means 24 also includes what one calls an input port, an input connector, etc. It does not matter whether the mode of the connection is wired or wireless.

The transmitting means 25 is connected to at least the light sources 12, which enables the light sources 12 to be controlled to be on, etc. based on the operation results obtained by the information processing unit 20. The transmitting means 25 also includes what one calls an output port, an output connector, etc. It does not matter whether the mode of the transmission is wired or wireless.

The light sources 12 are arranged in the vicinity of the tip of the rod-like main body 11. Any type of these light sources 12 becomes on, off, or on and off in an oral cavity according to the report to indicate, in the oral cavity, and report the state of the intraoral medical device to an operator. In this embodiment, LEDs are used as the light sources. More specifically, the white LED 12a, the red LED 12b, the green LED 12c, and the blue LED 12d are arranged.

The sensor 15 is arranged adjacent to the camera module 14, and measures the temperature of the camera module 14. The type of the sensor 15 is not particularly limited as long as the sensor 15 allows the obtained temperature to be acquired as digital data.

In this embodiment, the sensor to measure temperature is used as a sensor for grasping the condition for the intraoral medical device to appropriately carry out the function of the intraoral medical device. Not temperature but any other physical quantity may be obtained with a sensor as long as necessary for grasping the condition for the intraoral medical device to appropriately carry out the function thereof. Examples of such another sensor include sensors to detect contact, and pressing force.

### <Others>

Other than the above, in this embodiment, any component necessary for the digital impression device 1 is included. Examples of such components include ON and OFF switches for a power source, and switches for starting and stopping to obtain images.

While the digital impression device has been described in this embodiment as an example of intraoral medical devices, the intraoral reporting device of the present disclosure can be used for any intraoral medical device to be inserted in an oral cavity. Examples of such an intraoral medical device include an intraoral camera, an intraoral illuminator, and a periodontal pocket measurement instrument.

[Operation, Effects, etc. of Digital Impression Device]

### <Ordinary Operation>

In an ordinary scene where an intraoral shape is scanned with the digital impression device 1, the digital impression device 1 operates as follows.

An operator holds the intraoral scanner 10, inserts, into an oral cavity, the tip of the main body 11, which is on the side where the light sources 12 and the camera module 14 are arranged, and begins to photograph an area to be photographed. The operator obtains images of the area all over a site to be obtained while moving the camera module 14 along the area to be photographed.

At this time, the information processing unit 20 sends the light sources 12 the command that the light sources 12 are on, etc., which is suitable for the illumination to ordinarily obtain images. Specifically, the while LED 12a, the red LED 12b, the green LED 12c, and the blue LED 12d are emitted in turn continuously. Because the emitted LED is changed at high speed, white light illumination is visible to the naked eyes.

### <Intraoral Reporting Device>

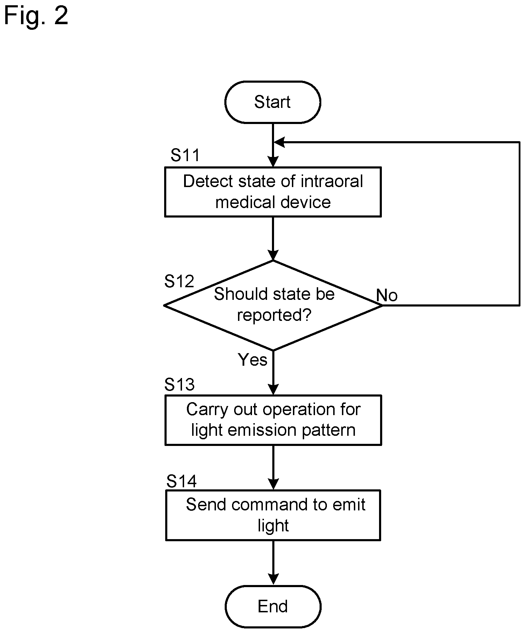

The intraoral reporting device operates during the above-described ordinary operation. During the operation of the intraoral reporting device, the light sources 12 become on, etc. as necessary. Fig. 2 shows the flow of the operation of the intraoral reporting device. As can be seen from Fig. 2, steps S11 to S14 are carried out in the intraoral reporting device. Hereinafter the steps will be each described.

In step S11, the state of the intraoral medical device is detected. Here, to detect the "state" is to detect that the intraoral medical device is in the state where the function thereof can be or cannot be maintained; or to detect whether or not the intraoral medical device is in the state where the function thereof cannot be maintained soon. In this embodiment, "scanning stops" is detected as the state where the function of the digital impression device 1 cannot be maintained, and "high temperature of intraoral scanner", "low temperature of intraoral scanner", or "almost limit of obtained data volume" is detected as the state where the function thereof cannot be maintained soon.

Concerning "scanning stops", the information processing unit 20 itself can determine the state where the information processing unit 20 does not obtain image information or, even if having obtained the information, cannot process the information, although the scanning is not intentionally stopped with a switch or the like. An example of the cause for the scanning to stop like this is the case where the operator moves the intraoral scanner 10 too fast.

"High temperature of intraoral scanner" and "low temperature of intraoral scanner" can be determined by the information processing unit 20 based on the information from the sensor 15.

Concerning "almost limit of obtained data volume", the information processing unit 20 itself can determine the data volume that can be stored further based on current stored data information.

In step S12, the information processing unit 20 determines whether to be reported based on the detected information obtained in step S11. If it is determined that there is no problem when the function is maintained, No is selected, and the flow returns to step S11. If it is determined that there is some problem when the function is maintained, Yes is selected, and the flow goes to step S13.

In step S13, the information processing unit 20 carries out operation to determine a report based on the detected information obtained in step S11, and in step S14, sends the command of the result to the light sources 12 to make the light sources 12 be on, etc. based on the command, which is specifically as follows in this embodiment.
"intraoral scanning stops": the red LED is on
"low temperature of scanner": the blue LED is on and off
"high temperature of scanner": the red LED is on and off
"almost limit of obtained data volume": the green LED is on and off for several seconds

For the light at this time, light (having luminance, lightness and/or color) sufficiently visible even in an oral cavity is preferably used. Thus, any color other than colors visible as white is preferably used.

As described above, the intraoral reporting device allows an operator to know the information on the state of the intraoral medical device (particularly, know that some problem has occurred) from the indication in an oral cavity, and can cognize this information without shifting their gaze from the oral cavity, which can prevent the operator from continuing operation without recognizing problems.

### [Reference Signe List]

1 digital impression device (intraoral medical device)
10 intraoral scanner
11 main body
12 light sources
13 reflecting mirror
14 camera module
15 sensor
20 information processing unit

## Claims

1. An intraoral reporting device to report a state of an intraoral medical device, the intraoral reporting device comprising:
an information processing unit; and
a light source connected to the information processing unit, and arranged so as to be able to emit light in an oral cavity, wherein
the information processing unit executes the process such that the light source is on, or on and off when detecting that the intraoral medical device is in a state where a function of the intraoral medical device is difficult to be maintained, or where the function thereof is difficult to be maintained soon.

2. An intraoral medical device comprising the intraoral reporting device according to claim 1.
